# EUROPÄISCHE PATENTSCHRIFT

(11) **EP 0 800 802 B1**
(45) Veröffentlichungstag und Bekanntmachung des Hinweises auf die Patenterteilung: **19.06.2002**
(21) Anmeldenummer: 97810040.2
(22) Anmeldetag: 28.01.1997
(51) Int. Cl.: A61F 2/30

(54) **Metallimplantat mit einer Oberfläche und Verfahren zum Herstellen der Oberfläche**
Metallic implant having a surface and method of producing the surface
Implant métallique comportant une surface et procédé pour fabriquer la surface

(30) Priorität: 10.04.1996 EP 96810221
(43) Veröffentlichungstag der Anmeldung: 15.10.1997
(73) Patentinhaber: Sulzer Orthopädie AG, 6340 Baar (CH)
(72) Erfinder: Baege, Roland, 8542 Wiesendangen (CH); Cassells, John Maclaren, Houghton, Huntingdon, PE 17 2BN (GB); King, Toby StJohn, Trumpington, Cambridge CB2 2HT (GB); Large, Timothy Andrew, Papworth Everard, Cambs CB3 8UQ (GB); Miller, Anne Tregoning, Cambridge CB2 2EZ (GB)
(74) Vertreter: Manitz, Finsterwald & Partner Gbr

(56) Entgegenhaltungen:
- EP-A- 0 373 011
- EP-A- 0 540 290
- DE-A- 3 119 130
- FR-A- 2 169 945
- US-A- 5 246 530
- US-A- 5 258 098
- US-A- 5 344 458

## Beschreibung

Die Erfindung handelt von einem Metallimplantat beispielsweise für Knochen mit einer Oberfläche, die eine Grobstruktur aus Erhöhungen und Vertiefungen aufweist und von Verfahren zum Herstellen der Oberfläche.

In der Teilanmeldung US-A-4,673,409 und der zugehörigen Hauptanmeldung US-A-4,608,052 wird eine Oberfläche gezeigt, bei der ausgehend von einer glatten Metalloberfläche Pfeiler erzeugt werden, indem mit einem Laser das Material um die Pfeiler herum in Form von Kanälen aus aneinandergereihten zylindrischen Sacklöchern verflüssigt und weggedampft wird. Diese Pfeiler bilden mit ihren Stirnflächen, die in der ursprünglichen glatten Metalloberfläche liegen, Abstützflächen für eine Primärverankerung im Knochengewebe und die sie umgebenden Hohlräume können später einwachsendes Knochengewebe oder Knochenzement aufnehmen. Diese Art der Metallbearbeitung ist enorm aufwendig, da das überflüssige Material bei dem geringen Wirkungsgrad eines Lasers verdampft werden muss und da der Focus der Laseroptik in seinem Abstand zur Oberfläche sehr genau nachgeführt werden muss. Im weiteren entstehen beim spontanen Erschmelzen und Verdampfen Metallspritzer, die in keiner Weise erwünscht sind, sodass in der US-A-5,246,530 vorgeschlagen wird, vor dem Laserbohren eine später entfernbare Schutzschicht aufzubringen, welche mit durchbohrt wird und welche ein Auftreffen von flüssigen Metallspritzern auf der von ihr abgedeckten Fläche verhindert. Als typische Schutzschichten werden lösungsmittellösliche Binder mit Metalloxiden angesehen, die bei hohen Temperaturen wärmebeständig sind. Da mit dieser Massnahme nicht verhindert werden kann, dass sich Metallspritzer in bereits gebohrten Sacklöchern auf dem Grundmaterial festsetzen, wird weiterhin vorgeschlagen zwischen der Schutzschicht und dem Grundmaterial eine säurefeste Zwischenschicht anzubringen, die ein Entfernen der Metallspritzer in den Bohrungen durch Aetzen erlaubt und die Bohrungen vergrössert.

Aufgabe der Erfindung ist es, Oberflächen für eine gute Verankerung eines Metallimplantates zu schaffen und gleichzeitig Verfahren zu entwickeln, die eine rationelle Herstellung solcher Oberflächen gestatten.

Diese Aufgabe wird für das Implantat mit den Kennzeichen vom unabhängigen Anspruch 1 dadurch gelöst, dass die Oberfläche von einem Netz von vorstehenden Rippen durchzogen ist, welche Knotenpunkte und Maschen mit einer Maschenweite von 2 mm bis 0,4 mm bilden, dass die Maschen jeweils eine Vertiefung in Form eines Ausschnitts aus einer Kugelkalotte bilden, und dass die Grobstruktur eine Feinstruktur für die Verankerung aufweist.

Eine solche Oberfläche hat den Vorteil, dass für die Primärverankerung ein Netzwerk von Rippen zu Verfügung steht, deren Kanten an den Kugelkalotten richtungsunabhängig Scherkräfte aufnehmen können und deren Kanten als Summe wegen der kleinen Dimension und wegen der dichten Anordnung der Kalotten eine enorme Länge zur Aufnahme von Scherkräften aufweisen.

Als Verankerungstiefe für einwachsendes Knochenmaterial ist eine Tiefe der Kalotten vorgesehen, die bis ein weniges über den Radius einer Halbkugelschale gehen kann.

Für die Primärverankerung von nicht zementierten Prothesen wird im Knochen ein Knochenbett ausgehöhlt, das einer geringfügig verkleinerten Hüllfläche an der Prothesenoberfläche entspricht. In einem solchen Fall ist es für die Primärverankerung weiterhin vorteilhaft, wenn die Rippen zwischen zwei Knotenpunkten einen Sattel bilden, weil sich die Kalotten an ihren Rändern teilweise durchdringen. Mit einer solchen Anordnung ist gewährleistet, dass mit einer Vergrösserung der Normalkraft zwischen Oberfläche und Knochenmaterial zunächst die Kanten an den Knotenpunkten und dann die Kanten an den Sattelpunkten in das Knochenmaterial eindringen und eine mit der Eindringtiefe sich sehr schnell vergrössernde Abstützfläche bilden, die nicht in der Umhüllenden in der Oberfläche liegt und die ein Gleiten verhindert. Wenn die Absenkung V der Sattelpunkte gegenüber den Knotenpunkten 0 < V < 50 % der Tiefe der Kalotten beträgt, kann eine Perforation der Knochenstruktur durch einzelne Pfeiler vermieden werden und es kann eine ausgeprägte Stirnfläche vermieden werden, die als Gleitfläche mit einer Umhüllenden zusammenfällt. Eine Oberfläche dieser Art eignet sich besonders als direkte Verankerung für ein Implantat im Knochen, wenn dieses aus körperverträglichem Material wie zum Beispiel Titan oder Titanlegierungen besteht. Bei Metallen wie z.B. Kobalt-Chrom-Legierungen kann diese Oberfläche für eine Verankerung in Knochenzement verwendet werden. Durch die netzförmige Anordnung der Rippen und durch deren Querschnittsvergrösserung mit wachsender Tiefe lassen sich auch im relativ weichen Reintitan filigrane Maschen mit einer Maschenweite bis zu 0,4 mm herunter einsetzen.

Die Kugelkalotten können mit ihren Mittelpunkten in Abständen angeordnet sein, die einem Raster entsprechen. Dies ist jedoch nicht zwingend. Da auch die gemischte Form mit schmalen Rippen ohne Sattelpunkte und mit Rippen mit Sattelpunkten als Grobstruktur geeignet ist, dürfen sich die Kugelkalotten auch in einer "wilden" Anordnung wenig oder gerade nicht mehr überschneiden. Ebenso können die Kugelkalotten in geringer Ueberschneidung auch wurmartig geschichtet oder aneinandergereiht sein, beispielsweise in Form von dicht aneinanderliegenden Spiralwindungen auf der Aussenseite einer Hüftgelenkschale oder in der Anordnung eines projezierten Tannennadelzweiges. Eine solche Grobstruktur der Oberfläche, auf der die Kugelkalotten wie ein Zweig aneinandergereiht sind, von dem seitlich Nadeln wegstehen, hat den Vorteil, dass einwachsendes Knochengewebe durch das Vorhandensein von wurmartigen Abschnitten auch längs der Achsen der wurmartigen Strukturen wegen der geringeren Rippenhöhe Blutgefässe zu seiner Versorgung aufbauen kann.

Vorteilhaft für die Verankerung ist, wenn auch auf gekrümmten oder geneigten Oberflächen die filigrane Netzstruktur als zusammenhängende Fläche rationell hergestellt werden kann. Dies geschieht entsprechend den Kennzeichen vom unabhängigen Anspruch 7 dadurch, dass Löcher mit einem kleineren Durchmesser als die Maschenweite resp. die späteren Kugelkalotten in einem Abstand der späteren Maschen im Schutzlack ausgespart werden, dass die mit Löchern im Schutzlack versehene Oberfläche zeitlich begrenzt einer elektrolytischen Flüssigkeit ausgesetzt wird, um durch elektrochemisches Abtragen oder Aetzen Ausschnitte von Kugelkalotten zu erzeugen, die durch schmale Rippen voneinander getrennt sind und eine grob strukturierte Zwischenoberfläche bilden, dass der restliche Schutzlack entfernt wird, und dass die grob strukturierte Oberfläche mit einer Feinstruktur, beispielsweise durch Sandstrahlen, versehen wird. Eine weitere rationelle Herstellung für eine Zwischenoberfläche ergibt sich aus den Kennzeichen des unabhängigen Anspruchs 9 dadurch, dass das Implantat zunächst mit einem haftenden, elektrochemisch wirksamen Schutzlack überzogen wird, der sich von einem Laserstrahl geringer Energiedichte verdampfen lässt, ohne dass das darunterliegende Metall davon tangiert wird. Im Gegenteil, bei genügend langwelligem Laserlicht kann die Reflexion des Metalls noch mit ausgenutzt werden, indem die Strahlung in den Schutzlack zurückgeworfen wird und indem weniger Strahlung vom Metall absorbiert wird. Ausserdem kann ein gegenüber der Metallbearbeitung wesentlich schwächerer und billiger Laser eingesetzt werden. Wegen dieser Energieschwelle zwischen Schutzlack und Implantat ist auch das Einhalten von einem bestimmten Fokussierabstand viel weniger heikel, um den Schutzlack abzutragen. Es können somit Löcher in dem Schutzlack erzeugt werden, ohne dass der Fokussierabstand genau eingehalten ist und mit einem Laserstrahl, der schräg zur Oberfläche steht. Mit dem Laserstrahl wird durch Relativbewegung zwischen Oberfläche und Laserkopf und durch wegabhängiges Auslösen vom Laserstrahl ein Feld von Löchern im Schutzlack erzeugt, wobei die Löcher den gleichen Mittenabstand wie die späteren Maschen haben, jedoch einen kleineren Durchmesser als die späteren Kugelkalotten aufweisen.

In einem weiteren, für alle Herstellverfahren gemeinsamen Schritt wird diese mit Löchern im Schutzlack versehene Oberfläche einer elektrisch leitenden Flüssigkeit, einem Elektrolyten, ausgesetzt, um mit deren Hilfe durch elektrochemisches Abtragen und/oder Aetzen schalenförmige Vertiefungen im Bereich der Löcher zu erzeugen. Ausgehend vom Durchmesser der Löcher im Schutzlack und von der Einwirkzeit beim elektrochemischen Abtragen und/oder Aetzen entstehen in die Tiefe und im Durchmesser wachsende Schalen. Es ist eine etwas erstaunliche Eigenschaft des Verfahrens, dass im Bereich der vorgegebenen Dimensionen für die Maschenweite und für die entsprechend kleineren runden Löcher im Schutzlack praktisch immer Vertiefungen in Form von Kugelkalotten entstehen, die sich je nach Einwirkzeit von einer flachen Kugelschale bis zu einer Halbkugelschale erweitern können, welche im besten Fall noch einen kleinen zylindrischen Rand aufweist. Eine mögliche Erklärung für diesen Effekt sind Diffusionsvorgänge, wenn nur relativ kleine Löcher im Schutzlack vorhanden sind. Der Mittenabstand der Löcher, der Durchmesser der Löcher und die Einwirkzeit beim Abtragen werden so gewählt, dass beim Abbrechen des Abtragens Rippen mit einer gewünschten Wandstärke zwischen zwei benachbarten Kugelkalotten stehen bleiben und eine gewünschte Schalentiefe erreicht wird. Dieser Vorgang hat den Vorteil, dass die Metallbearbeitung zur Erzeugung dieser Zwischenform der Oberfläche über grosse Oberflächenbereiche gleichzeitig und innerhalb weniger Minuten erfolgen kann. Nach dem Entfernen des elektrochemisch wirksamen Schutzlacks besteht eine Zwischenoberfläche, die durch Sandstrahlen eine solche Feinstruktur erhält, dass die oben wie im Anspruch 1 beschriebene Topographie entsteht. Bei geringfügiger Ueberschneidung der Kugelkalotten entstehen an den engsten Stellen der Rippen Sättel, während an den Knotenpunkten, wo mehrere Rippen zusammenlaufen, Gipfel stehen bleiben. Je nach Art des Schutzlacks, kann dieser beim Grobstrahlen gleich mitentfernt werden.

Aus der obigen Beschreibung ergibt sich, dass das Verfahren soweit abgewandelt werden kann, dass durch das kontinuierliche Wegverdampfen von Schutzlack längs einem vorgegebenen Linienabschnitt mit einem Laser eine Furche im Schutzlack erzeugt wird, die beim nachfolgenden elektrochemischen Abtragen eine entsprechend breitere Rinne im Metall erzeugt, deren Kontur im Querschnitt im wesentlichen dem Ausschnitt eines Kreises entspricht. Letzteres solange die Rinnen eine Breite von 2 mm bis 0,4 mm aufweisen und die Furchen in einer Breite, die einem Bruchteil der Breite der Rinnen entspricht, vom Laser geschrieben werden. Der Vorteil eines solchen Verfahrens besteht darin, dass der Laserstrahl auf beliebigen gekrümmten oder unterbrochenen Linien entlang einer mit Schutzlack abgedeckten Metalloberfläche wie der Stichel eines Graveurs geführt werden kann, um Muster zu erzeugen, die sich nachfolgend durch das kontrollierte elektrochemische Abtragen oder Aetzen auf der Metalloberfläche als eine Grobstruktur abbilden. Die zwischen den Rinnen stehengebliebenen Rippen bilden Verankerungsflächen, die quer zu möglichen Scherbelastungen in der Oberfläche wirksam sein können. Eine Bedingung beim Schreiben des Lasers ist, dass die Schreibgeschwindigkeit und die Intensität des Lasers zusätzlich aufeinander abgestimmt sein müssen. Eine weitere Bedingung für den Schutzlack und die Intensität beim elektrochemischen Abtragen oder Aetzen ist die, dass die Intensität nur so stark gewählt werden darf, dass der im Bereich der Furche nur einseitig abgestützte Schutzlack während seiner Unterwanderung beim elektrochemischen Abtragen nicht ausbröckelt, um eine reproduzierbare Grobstruktur zu schaffen. Nach dem Entfernen des Schutzlacks besteht eine Grobstruktur der Oberfläche mit Rinnenabschnitten, die eng geschichtet sein können und die sich gegenseitig durchdringen können. Dabei können ebenfalls auf einer bestimmten Oberfläche Rippen erzeugt werden, die für eine Verankerung eines Metallimplantats geeignet sind, die jedoch bei gleichen kleinsten Abmessungen der Kavitäten gegenüber den Kugelkalotten eine geringere Summe ihrer Kantenlängen aufweisen. Im Extremfall kann so ein Gitter von sich überschneidenden Rinnen erzeugt werden, bei dem die Rippen zu vorstehenden Stiften degeneriert sind, deren Summe ihrer Kantenlängen einem Minimum entspricht.

Als elektrochemisch wirksamer Schutzlack, der sich auf Metalle auftragen, mit einem Laser bohren und nur im Bereich der gebohrten Löcher von einer elektrochemischen Flüssigkeit durchdringen lässt, kommen viele Produkte in Frage. Für die Auswahlversuche war jedoch entscheidend, dass aus wirtschaftlichen Gründen bezüglich Erhaltung der Sauberkeit beim Herstellen der Einwachsoberfläche keine weiteren formgebende Fertigungsoperationen am Implantat mehr folgen sollten. Der elektrochemisch wirksame Schutzlack sollte daher auch in der Lage sein, scharfkantige Flächenübergänge, wie sie an Rippen oder beispielsweise an Verklinkungen von Hüftgelenkschalen vorkommen, wirksam abzudecken. Entsprechende Produkte sind als Schutzlack in den Ausführungsbeispielen erwähnt. Besonders geeignet ist ein Schutzlack, wenn bei guter Haftung nur eine geringe Schichtdicke zur Isolation notwendig ist, da dann auch bei scharfkantigen Uebergängen wegen der dünnen Schichtdicken eine Mindestschichtdicke einfacher einzuhalten ist. Ein weiterer Vorteil bei dünnen Schutzlackschichten ist, dass die beim elektrochemischen Abtragen und/oder Aetzen entstehenden Gasblasen weniger die Tendenz haben, in den Löchern vom Schutzlack hängen zu bleiben und so den Abtragsprozess zu hemmen.

Weitere vorteilhafte Verbesserungen werden erzielt, wenn die elektrolytisch abtragende Flüssigkeit innerhalb eines Bades als unverbrauchter Strahl quer über die Oberfläche zugeführt wird und gleichzeitig die entstehenden Gasblasen fortschwemmt. Bei Salzlösungen wird das Implantat als Elektrode verwendet und sollte in einem nicht benetzten Bereich kontaktiert werden. Eine entsprechende Gegenelektrode sollte in einem Abstand von weniger als 10 mm zur zu bearbeitenden Oberfläche angebracht werden. Die Spülung erfolgt dann beispielsweise mit einem Strahl in den Spalt zwischen den beiden Elektroden. Eine andere Möglichkeit sind Elektroden, die im Arbeitsspalt ein Lochbild aufweisen, durch welches frischer Elektrolyt eingespritzt wird. Ueber die Dauer der angelegten Spannung kann der Abtrag sehr genau dosiert werden, wobei durch das Einhalten einer bestimmten Temperatur des Elektrolyten dessen Leitfähigkeit und der Stromfluss genauer einhaltbar sind. Bei bestimmten Metallen wie beispielsweise Titan können sich passivierende Schichten bilden, die eine höhere Anfangsspannung als für den eigentlichen Abtrag bei Spannungen zwischen 15 bis 60 Volt notwendig machen können. Für Titan hat sich in Verbindung mit einer Kochsalzlösung eine Spannung von 40 Volt bewährt.

Im folgenden wird die Erfindung anhand von Ausführungsbeispielen beschrieben. Es zeigen:
- Figur 1a: Schematisch die vergrösserte Ansicht eines Ausschnitts einer erfindungsgemässen Oberfläche mit sich überschneidenden Kugelkalotten;
- Figur 1b: schematisch einen vergrösserten Ausschnitt aus Figur 1a;
- Figur 1c: schematisch eine Darstellung mit Höhenkurven für einen quadratischen Raster gemäss Figur 1a und 1b;
- Figur 1d: schematisch eine Darstellung mit Höhenkurven gemäss Figur 1c, jedoch für einen Raster der aus gleichseitigen Dreiecken zusammengesetzt ist;
- Figur 1e: schematisch eine Maske, die schrittweise verschiebbar ist, um die späteren für das Aetzen notwendigen Löcher von einer durch Strahlung ausgelösten Verfestigung auszuschliessen;
- Figur 2: schematisch eine vergrösserte Ansicht einer Zwischenoberfläche, bei der Kugelkalotten in einem Raster analog zu Fig. 1d angeordnet sind;
- Figur 3: schematisch einen vergrösserten Schnitt für eine Zwischenoberfläche gemäss Figur 1a;
- Figur 4a: schematisch die vergrösserte Draufsicht auf eine Oberfläche, die mit Schutzlack beschichtet ist, wobei im Schutzlack bereits die Löcher für das elektrochemische Abtragen erzeugt sind;
- Figur 4b: schematisch einen Schnitt durch eine Oberfläche gemäss Figur 4a, bei der jedoch der Durchmesser der Löcher im Schutzlack grösser gewählt wurde, um flachere Kugelkalotten zu erzeugen;
- Figur 4c: schematisch einen Schnitt durch eine Oberfläche gemäss Figur 4a, bei der jedoch der Durchmesser der Löcher im Schutzlack kleiner gewählt wurde, um tiefere Kugelkalotten zu erzeugen;
- Figur 5: schematisch einen vergrösserten Schnitt durch eine gestufte Oberfläche, die mit Schutzlack beschichtet ist, wobei im Schutzlack bereits Löcher für den elektrochemischen Abtrag erzeugt sind;
- Figur 6: schematisch einen vergrösserten Schnitt durch eine zu einem Laserstrahl schräg stehende Oberfläche, die mit Schutzlack beschichtet ist, wobei im Schutzlack bereits Löcher für den elektrochemischen Abtrag erzeugt sind;
- Figur 7a: schematisch die Fixierung eines Femurschaftes für eine Beschichtung mit Schutzlack;
- Figur 7b: schematisch die Fixierung eines Femurschaftes beim elektrochemischen Abtragen;
- Figur 7c: schematisch eine Elektrode mit einem Lochbild zum Einbringen des Elektrolyten in den Abtragsspalt;
- Figur 8a: schematisch die vergrösserte Ansicht einer Hüftgelenkschale, an der eine Aufteilung der Abstände für die Löcher im Schutzlack auf zwei umlaufenden Bändern gezeigt ist;
- Figur 8b: schematisch eine Anordnung zum Herstellen der Löcher im Schutzlack einer Hüftgelenkschale gemäss Figur 8a mit einem Laser;
- Figur 8c: schematisch eine weitere Anordnung mit einem Laser zum Erzeugen von Löchern an einer mit Schutzlack beschichteten Hüftgelenkschale;
- Figur 9: schematisch die elektrochemische Bearbeitung an einer Aussenschale nach Figur 8a, b, c;
- Figur 10: eine vergrösserte Oberfläche an einem Implantat aus Metall mit sich nicht überschneidenden Kugelkalotten;
- Figur 11: schematisch einen vergrösserten Schnitt unterhalb der Hüllkurve eines Implantats, bei dem ineinandergewundene Rinnen mit dünnen Zwischenrippen in einer orthogonalen Anordnung erzeugt werden, und
- Figur 12: schematisch einen vergrösserten Schnitt unterhalb der Hüllkurve eines Implantats, bei dem Rinnen, die durch schmale Rippen getrennt sind, tannenbaumähnliche Strukturen in Form eines sechseckigen Flächenelementes bilden.

In den Figuren ist eine Oberfläche 2 für ein Metallimplantat 1 gezeigt, die eine Grobstruktur aus Erhöhungen 3 und Vertiefungen 4 aufweist, wobei die Oberfläche 2 von einem Netz 5 von vorstehenden Rippen 6 durchzogen ist, welche Knotenpunkte 7 und Maschen 8 mit einer Maschenweite von 2 mm bis 0,4 mm bilden, während die Vertiefungen Ausschnitte von Kugelkalotten 11 darstellen. Die Knotenpunkte 7 der Rippen können wie Berggipfel am weitesten vorstehen, während Rippen 6 die zwei Knotenpunkte 7 verbinden, jeweils einen tieferliegenden Sattel 10 bilden, wenn sich die Kugelkalotten 11 geringfügig durchdringen. Durch Abdecken mit einem elektrochemisch resistenten Schutzlack, in welchen beispielsweise mit einem Laser ohne das Metall zu verletzen, Löcher in einem vorgesehenen Abstand geschossen werden, kann auf wirtschaftliche Weise mittels elektrochemischen Abtragen eine grobstrukturierte Zwischenoberfläche geschaffen werden, die durch Sandstrahlen eine Feinstruktur erhält.

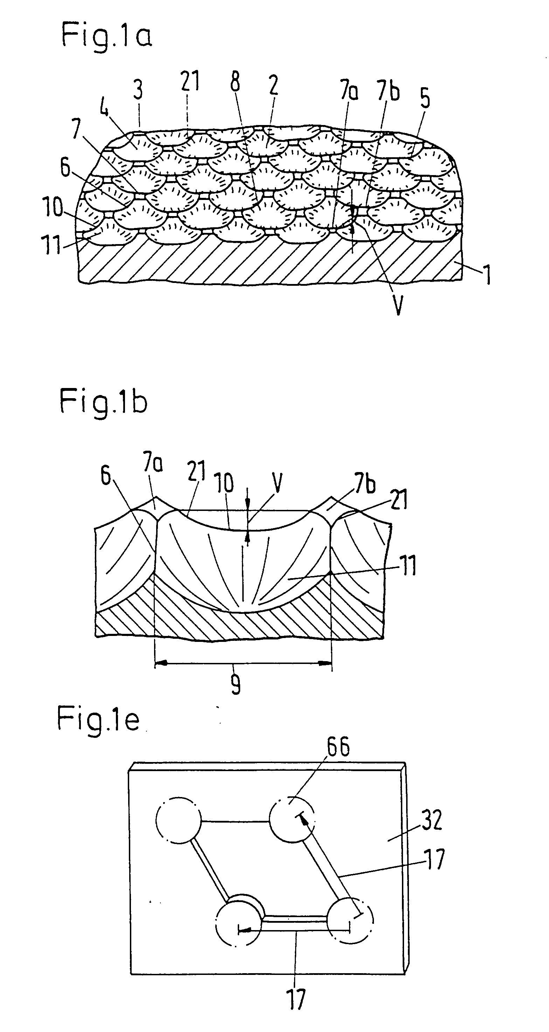

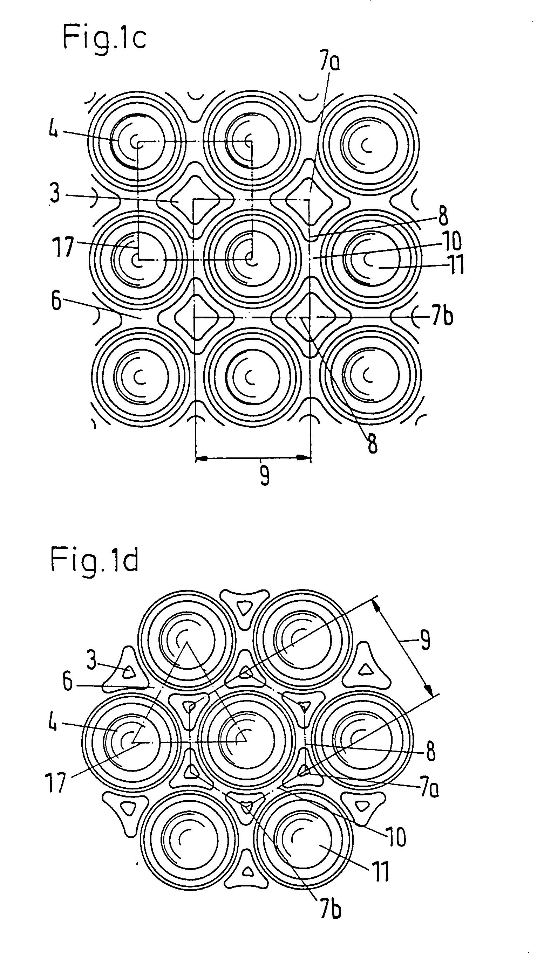

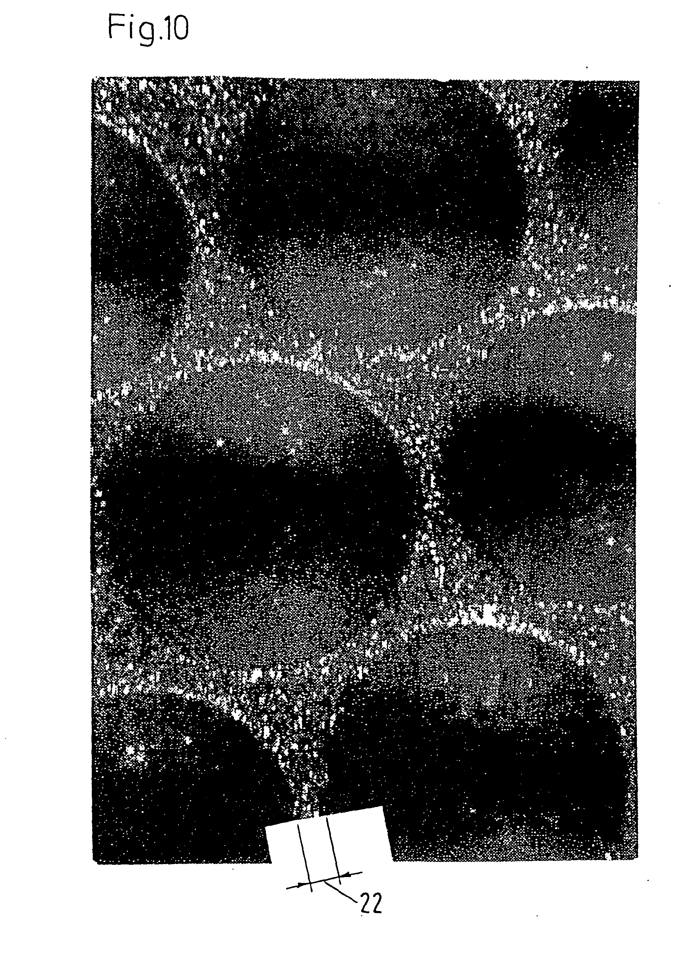

In den Figuren 1a, b, c ist die fertig bearbeitete Oberfläche 2 eines Knochenimplantats 1 dargestellt, die Erhöhungen 3 und Vertiefungen 4 aufweist. Die Erhöhungen 3 bestehen aus einem Netz 5 von vorstehenden Rippen 6, welche Knotenpunkte 7 und Maschen 8 mit einer Maschenweite 9 von 2 mm bis 0,4 mm aufweisen und Kanten 21 besitzen. Die Mittelpunkte der Maschen 8 sind bei diesem Ausführungsbeispiel in einem quadratischen Raster angeordnet, während sie im Beispiel von Figur 1d in Abständen 17 mit einem Raster aus gleichseitigen Dreiecken aufgebaut sind, damit die Rippen 6 unabhängig von ihrer Richtung jeweils gleiche Beschaffenheit haben und gleiche Längen aufweisen. Die Vertiefungen 4 sind aus Ausschnitten von Kugelkalotten 11 gebildet, deren Achsen durch die Mittelpunkte der Maschen 8 gehen. An ihren Knotenpunkten 7 stehen die Rippen 6 wie Gipfel 7a, 7b am weitesten vor, während sie als Verbindung von zwei solchen Knotenpunkten 7a, 7b einen tiefer liegenden Sattel 10 bilden, der um einen Betrag V tiefer als die Gipfel 7a, 7b angeordnet ist. Solche Sättel 10 entstehen, wenn sich die Kugelkalotten etwas überlappen. Dies muss jedoch nicht so sein, denn auch wenn sich die Kugelkalotten 11 gerade nicht mehr überlappen bleibt ein filigranes Netz 5 von Rippen 6 stehen. Figur 10 zeigt die Vergrösserung einer fotografierten Oberfläche mit Abständen 17 in einem Raster aus gleichseitigen Dreiecken analog zu Figur 1d, jedoch ohne Ueberlappung der Kugelkalotten. Die Maschenweite 9 beträgt hier 1,0 mm und die minimale Breite der Rippen beträgt etwas weniger als 0,1 mm. Die Kugelkalotten 11 besitzen einen Durchmesser von etwas mehr als 0,9 mm und weisen eine Tiefe 12 von 0,4 mm auf. Obwohl es sich in Figur 10 um ein Implantat aus relativ weichem Titan handelt, ist eine Wandstärke 22 von ≈ 0,1 mm am engsten Querschnitt der Rippen für eine Verankerung ausreichend, da der Querschnitt von der minimalen Breite weg weg in die Tiefe und längs der Rippe wächst. Diese Wirkung wird noch verstärkt, wenn man die Abstände 17 in einem quadratischen Raster wie im Beispiel der Figuren 1a, b, c wählt.

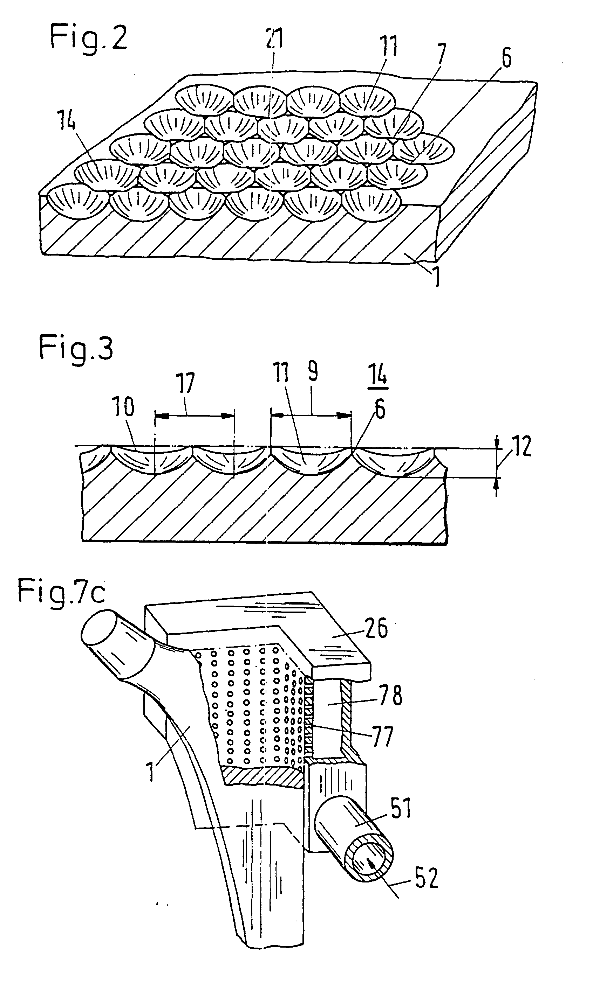

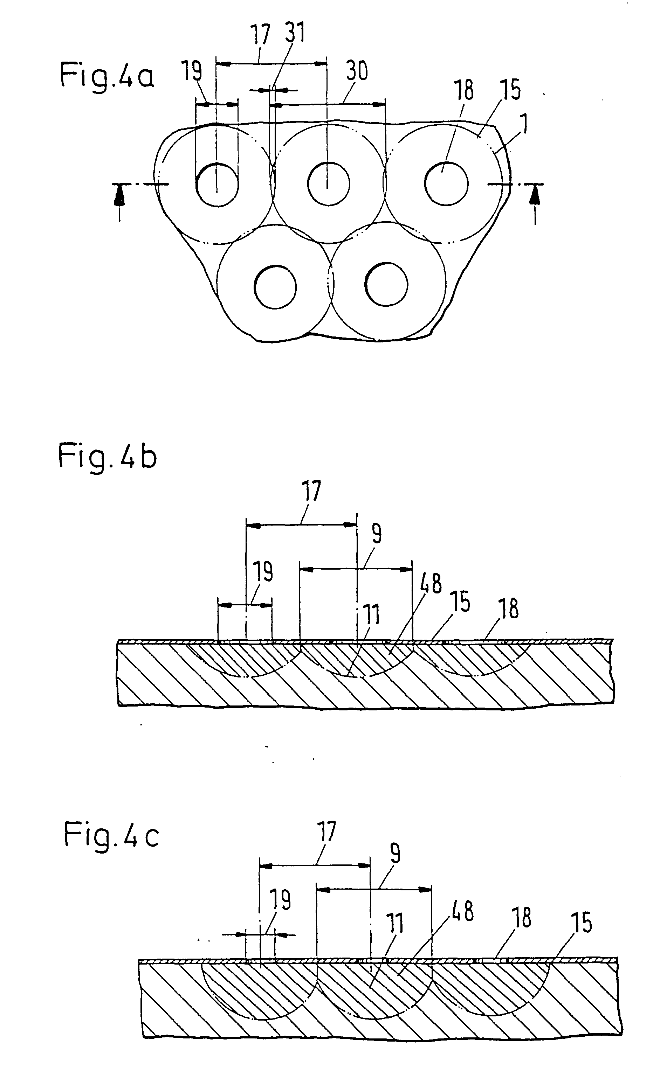

In den Figuren 2 und 3 ist eine Zwischenoberfläche 14 als Herstellungsvorstufe zu einer Oberfläche 2 gemäss einem Abstand 17 der Kugelkalotten 11 in einem Dreieckraster von Figur 1d dargestellt, deren Herstellung nachfolgend beschrieben ist. Als Zwischenoberfläche werden Kugelkalotten 11 mit einem Durchmesser 30 erzeugt, der grösser ist als der Abstand 17 ihrer Mittelpunkte, um im Rahmen der möglichen Herstellgenauigkeit durch die Ueberlappung Rippen 6 mit einem Sattel 10 stehen zu lassen. Dazu wird die ursprünglich glatte Ausgangsfläche gemäss Figuren 4a, 4b, 4c derart mit einem elektrochemisch wirksamen Schutzlack 15 abgedeckt, dass Aussparungen in Form von Löchern 18 mit einem Durchmesser 19, der wesentlich kleiner als der Durchmesser 30 der Kugelkalotten 11 ist, in einem Raster mit Abstand 17 erzeugt werden. Anschliessend wird die mit Löchern 18 im Schutzlack versehene Oberfläche einem Elektrolyten ausgesetzt, wobei im Bereich der Löcher ein elektrochemisches Abtragen oder Aetzen stattfindet. Je nach Untermass der Löcher 18 mit Durchmesser 19 gegenüber der angestrebten Durchmesser 30 der Kugelkalotten 11 muss die Einwirkzeit beim elektrochemischen Abtragen angepasst werden. Bei kleinem Untermass (Figur 4b) ergeben sich kurze Einwirkzeiten und flachere Kugelkalotten 11. Bei grossem Untermass (Figur 4c) ergeben sich längere Einwirkzeiten und tiefere Kugelkalotten 11. Als Grenzwert für eine beherrschbare Tiefe 12 der Kugelkalotten, kann man 60 % vom Durchmesser einer Halbkugel annehmen. In einem derartigen Fall verlaufen die ersten 10 % der Tiefe bis zur Halbkugelschale annähernd zylindrisch. Nach dem elektrolytischen Bearbeiten ist abgesehen vom haftenden Schutzlack 15 die Zwischenoberfläche 14 vorhanden. Je nach Art des Schutzlacks kann dieser durch ein Lösungsmittel, chemisch, durch Wärmeeinwirkung und/oder mechanisch entfernt werden, um zu einer Zwischenoberfläche 14 zu kommen, welche durch Sandstrahlen eine zusätzliche Feinstruktur erhält. Wenn der Schutzlack 15 mechanisch durch Sandstrahlen entfernbar ist, kann dessen Entfernung und die Erzeugung der endgültigen Struktur in einem Arbeitsgang erfolgen.

Das Aussparen der Löcher resp. das Verfestigen des Schutzlacks ausserhalb der Löcher 18 kann auch durch photochemische Effekte erfolgen, indem beispielsweise der Schutzlack durch Bestrahlung durch eine Maske ausserhalb der Löcher 18 verfestigt wird und im Bereich der nicht "belichteten" Löcher ausgewaschen wird, um so die Voraussetzungen für den elektrochemischen Abtrag zu erreichen. Eine für Figur 1d geeignete Maske 32 ist in Figur le gezeigt, wobei deren Verschiebewege zwischen der Bestrahlung in einem Raster mit Abständen 17 angedeutet sind. Als Schutzlack kommen Polymere in Frage, die sich durch Photoeffekt verfestigen lassen.

Eine andere Möglichkeit besteht darin, den Schutzlack 15 ganzflächig aufzutragen und sich verfestigen zu lassen, um anschliessend den Schutzlack im Bereich der Löcher 18 mit einem Laser wegzuverdampfen. Dabei ist es vorteilhaft, wenn das Metall im Bereich der Löcher weder verflüssigt noch verdampft wird.

### Beispiele:

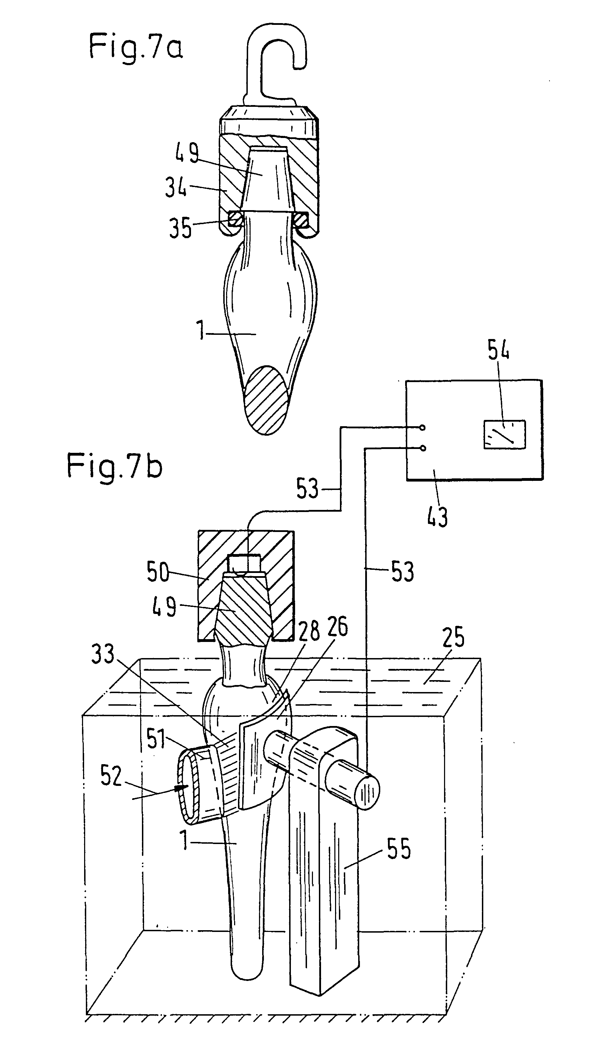

Bei der Verwendung des Schutzlacks Parylene ™, Poly-para-xylylene der Firma Union Carbide, welcher von der Nova Tran Ltd., precision surface polymers, Northampton, UK, vertrieben wird, geschieht das Auftragen im Vakuum durch Verdampfen von Parylene ™, welches auf den Oberflächen kondensiert und polymerisiert. Die Teile werden an Aufhängevorrichtungen, wie beispielsweise in Figur 7a, in einem Vakuumofen plaziert, wo das Parylene ™ bei 600°C verdampft wird. Die abgelagerte Schicht wächst mit etwa 2 µm pro Stunde, wobei auch schlecht zugängliche Bereiche gut abgedeckt werden. Als isolierende Schichtdicke S für das elektrochemische Abtragen genügt eine Dicke 3 µm < S < 10 µm, wobei Schichtdicken bis zu 100 µm möglich sind. Als sichere Schichtdicke, bei der auch scharfkantige Uebergänge noch genügend abgedeckt werden, hat sich eine Dicke S von 7 µm bewährt. Bei Dicken unter 5 µm könnte die Schutzschicht an bestimmten Orten durch einen Spülstrahl 33 (Figur 7b) beim elektrochemischen Aetzen abgeschält werden. Beim Aufbringen im Vakuum sind Dicken über 10 µm wenig wirtschaftlich.

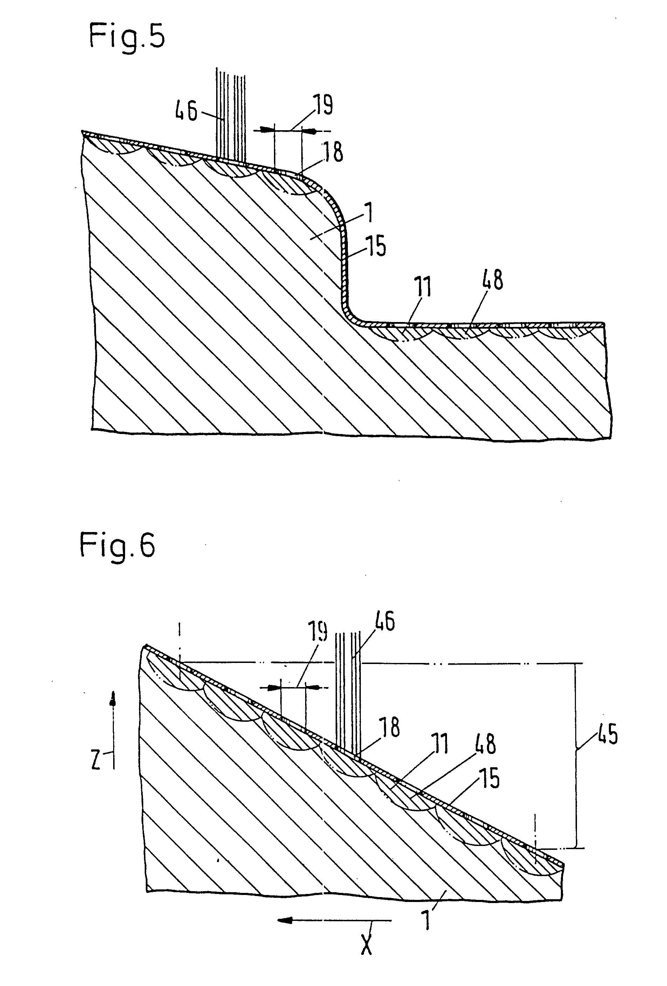

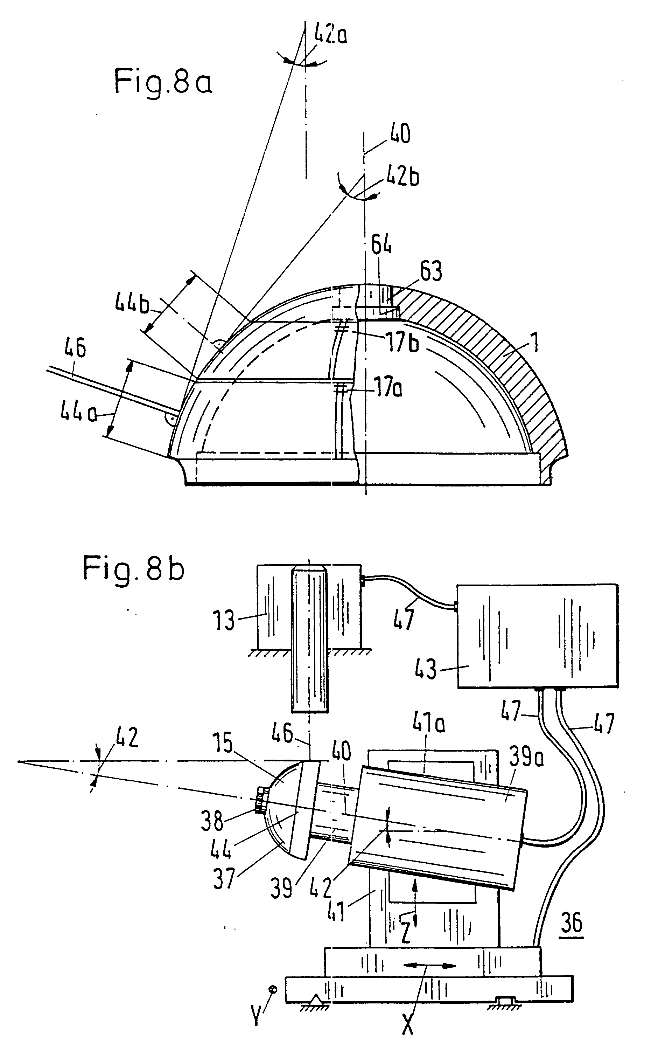

In einer Anordnung nach Figur 8b erscheint das Parylene ™ als dünner transparenter Schutzfilm auf dem Implantat, welches zur Erzeugung der Löcher 18 in einer numerisch gesteuerten Aufspannvorrichtung 36 aufgespannt wird, um mit einem Laser 13 Löcher 18 in den Schutzlack 15 zu bohren. In Figur 8b ist eine Hüftgelenkschale 37 mit einer Befestigungsschraube 38 auf einer drehbaren Spindel 39 befestigt, wobei die Spindelachse 40 mit der Polachse der Hüftgelenkschale zusammenfällt. Die Aufspannvorrichtung 36 besteht aus einem Ständer 41, der auf einem horizontal in Richtung x und y beweglichen Kreuztisch steht und einen Vertikalschlitten für die Bewegungsrichtung z aufnimmt. Senkrecht zum Kreuztisch ist ein Laser 13 angeordnet, der über eine Steuerung 43 mit der numerisch gesteuerten Aufspannvorrichtung 36 so koordiniert ist, dass er nach einem vorgegebenen Raster ein Lochbild in den Schutzlack schiesst. Die Steuerung 43 ist dazu mit Verbindungsleitungen 47 mit dem Laser 13 mit der Aufspannvorrichtung 36 und mit dem Spindelantrieb 39a der Spindel 39 verbunden, welche in einem Schwenkwinkel 42 am Vertikalschlitten 41a befestigt ist. In Figur 8a besteht die zu bearbeitende Oberfläche aus zwei Bändern 44a und 44b, die parallel zum Aequator umlaufen. Da entsprechend Figur 6 die Nachstellung vom Focus für ein Loch 18 innerhalb von Tiefenänderungen 45 mit einem Betrag < 8 mm nicht notwendig ist, können wie in den Figuren 5 und 6 dargestellt, die Löcher 18 in gestuften oder zum Laserstrahl 46 schräg stehenden Flächen innerhalb der zulässigen Tiefenänderung 45 schon durch Verschiebung mit dem Kreuzschlitten in einer x-y-Ebene erzeugt werden. Dadurch, dass der Schwenkwinkel 42, 42a, 42b, mit einer Tangente an ein Band 44, 44a, 44b gebildet wird, welche parallel zum Kreuztisch verläuft wird ein trapezförmiges Lochbild von einem Raster 17 in den Koordinaten des Kreuztisches mit nur geringen Verzerrungen 17a, 17b und ohne Bewegung in der Achse z abgebildet. Die Spindel 39 wird jeweils um eine bestimmte Teilung weitergedreht, sodass auf dem Umfang des Bandes 44 trapezförmige, leicht verzerrte Lochbilder aneinandergereiht werden. Es ist ebenso möglich, die Drehspindel 39 mit einer numerisch gesteuerten Feinverstellung zu versehen und ein Lochbild mit einem Programm für Spindelantrieb 39a und für Verschiebungen in der X-Achse herzustellen.

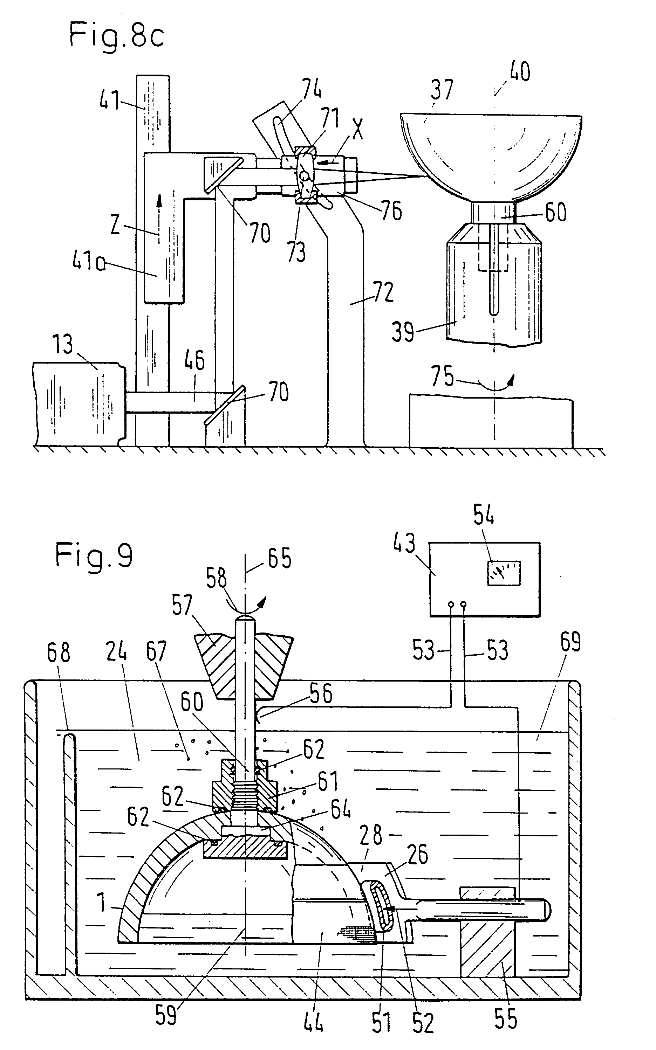

Eine weitere Möglichkeit zum teilweise Kompensieren der Tiefenverstellung gegenüber einem horizontalen Laserstrahl 46 ist in Figur 8c gezeigt. Der Laserstrahl 46 ist über zwei 45 Grad-Spiegel 70, von denen einer über einen Vertikalschlitten 41a an einem Ständer 41 verstellbar angeordnet ist, in der Vertikalen verschiebbar. Der parallel austretende Laserstrahl wird mit einer Linse 71 fokussiert, die ihrerseits an einem Horizontalschlitten 76 fixiert gegenüber dem Vertikalschlitten 41a horizontal verschiebbar ist. Der Laserstrahl ist auf die Oberfläche einer Hüftgelenkschale 37 fokussiert, die ihrerseits mit einem Spannbolzen 60 in einer numerisch gesteuerten Drehspindel 39 so fixiert ist, dass Polachse und Spindelachse 40 zusammenfallen. Durch eine gesteuerte Drehbewegung 75 können alle Punkte, die auf dem gleichen Breitenkreis liegen, angefahren werden, während zur Verschiebung des Brennflecks längs eines Meridians der Vertikalschlitten 41a numerisch gesteuert in Richtung Z vertikal verschoben wird und gleichzeitig eine Korrektur des Linsenabstandes in der Horizontalen vorgenommen wird. Diese Korrektur passiert hier zwangsläufig mit der Höhenverstellung des Vertikalschlittens 41a, dadurch, dass der Horizontalschlitten 76 mit einem Zapfen 73 in einer Führungsnut 74 geführt ist, die zum Meridian der Hüftgelenkschale 37 annähernd parallel verschoben ist. Die Führungsnut 74 ist in einem ortsfesten Ständer 72 eingearbeitet. Sie muss nicht genau parallel zur Oberfläche des Werkstücks verlaufen, da der Abstand des Brennflecks im Bereich von bis zu 8 mm schwanken darf, um genügend genaue Löcher in den Schutzlack zu schiessen.

In den Figuren 5 und 6 sind die Löcher 18 mit Durchmesser 19 sowie die später erst durch elektrochemisches Abtragen zu erzeugenden Kugelkalotten 11 -letztere durch eine engere Schraffur 48 - angedeutet.

Weitere Beispiele für das Aufbringen von Schutzlack:
Andere Arten von Schutzlack 15 können nach unterschiedlichen Methoden auf das Implantat 1 aufgebracht werden
   - ein in Methanol lösliches Phenolharz wie beispielsweise das Produkt Chemlock 104 ™ der Firma Henkel lässt sich durch Tauchlackieren aufbringen,
   - phenolhaltige Epoxidharze wie das Produkt Goldlacquer ™ der Firma Holden oder Cascophen ™ der Firma Boden lassen sich durch Spraylackieren aufbringen,
   - eine weitere Möglichkeit besteht im elektrostatischen Pulverbeschichten,
   um ähnliche Bedingungen wie in den Figuren 5, 6, 8a, 8b dargestellt für das Bohren von Löchern 18 im Schutzlack 15 mit Laserstrahlen zu schaffen, ohne das darunterliegende Metall zu verletzen.

Eine in Figur 8a dargestellte Ausschenschale 1 für ein künstliches Hüftgelenk lässt sich an einer gestuften Aufnahmebohrung 63 polseitig zum Aufbringen von Schutzlack befestigen, wobei eine in Figur 9 gezeigte Schulter 64 keinen Schutzlack erhält, damit ein metallischer Kontakt für eine Stromübergabe möglich ist. Diese Aufnahmebohrung 63 mit Schulter 64 dient gleichzeitig als Befestigungsfläche und Gegenfläche für die Befestigungsschraube 38 in Figur 8b beim Laserbohren vom Schutzlack, während in Figur 9 ein Spannbolzen 60 mit Gegenmutter 61 so angeordnet ist, dass über Weichdichtungen 62 die blanken, für die Zentrierung und die Stromübergabe vorgesehenen Stellen gegen den Kontakt mit dem Elektrolyten 24 abgedichtet sind.

In der Anordnung von Figur 9 ist eine auf dem Spannbolzen 60 befestigte Aussenschale 1 mit einem Lochbild 44 zu sehen. Der Spannbolzen 60 ist in einer langsam drehenden, isolierenden Spannzange 57 eingespannt, derart, dass die Lagerschale 1 um ihre Polachse 59 dreht, wobei die Drehung 58 wenige Umdrehungen pro Minute beträgt. Typisch sind z.B. 5 Umdrehungen pro Minute. Die Lagerschale 1 dreht in einem Elektrolytbad 69, welches einen Ueberlauf 68 besitzt. Eine Steuerung 43 schliesst die Lagerschale 1 über eine Zuleitung 53, einen Schleifkontakt 56 und über den Spannbolzen 60 an ein elektrisches Potential an. Eine Gegenelektrode 26 ist über eine Zuleitung 53 an ein weiteres Potential angeschlossen, dessen Differenz beispielsweise über eine Anzeige 54 an der Steuerung 43 ablesbar ist. Die Gegenelektrode 26 ist an einem Ständer 55 befestigt und in Form einer Flachelektrode um einen Sektor der Lagerschale 1 herumgeführt, damit im Bereich des Lochbildes 44 ein Kanal mit einer Weite unter 10 mm entsteht, in welchen frischer Elektrolyt 52 beispielsweise eine Kochsalzlösung über ein abgeflachtes Spülrohr 51 eingespritzt wird. Mit dem Anlegen einer passenden Spannung zwischen den Elektroden findet im Bereich der im Schutzlack angebrachten Löcher ein elektrochemischer Metallabtrag statt. Die Metallionen gehen in Lösung und bilden Salze, während gleichzeitig Gasblasen 67 entstehen, die den Vorgang hemmen könnten, wenn sie nicht aufgrund ihrer Entstehungsdynamik und mit dem Spülstrahl aus dem Arbeitsbereich weggeschwemmt würden. Durch eine Strombegrenzung und eine Ueberwachung der Einschaltzeit lässt sich die Grösse der entstehenden Kugelkalotten 11 sehr genau einhalten.

Ein weiteres Beispiel für einen Prothesenschaft 1 ist in den Figuren 7a, 7b gezeigt. Der Prothesenschaft ist in Figur 7a mit seinem Konus 49 in einer Aufhängevorrichtung 34 befestigbar, um den Schutzlack anzubringen. Ein Dichtring hält den Konus 34 frei von Schutzlack, damit dieser nach dem Bohren der Löcher 18 im Schutzlack 15 gemäss Figur 7b für die Befestigung in einem Elektrodenhalter 50 verwendet werden kann. Die Stromübergabe erfolgt über einen federnden Kontakt auf der Stirnseite vom Konus 34. Der Prothesenschaft 1 ist ortsfest im Elektrodenhalter 50 befestigt und steht in einem Elektrolytbad, einer Kochsalzlösung 25, zusammen mit einer Gegenelektrode 26, die als Flachelektrode der Kontur des Schaftes folgt und einen Kanal 28 bildet, in welchen über ein Spülrohr 51 gereinigter Elektrolyt in einem Spülstrahl 33 eingespritzt wird. Dabei beträgt die Durchflussmenge 10 l/min, und mehr, wenn sich der Kanal 28 über eine Oberfläche von 15 cm² am Werkstück erstreckt. Dies entspricht etwa einer Geschwindigkeit von 400 mm/s im Kanal 28. Eine typische Anordnung für eine Elektrode wäre eine Flachelektrode die einen Abstand von 2 mm zum Bohrbild aufweist und deren Kanten in jeder Richtung um etwa 4 mm gegenüber dem Bohrbild gekürzt sind, wenn mit einer gesättigten Kochsalzlösung gearbeitet wird.

Die Stromdichte sollte generell zwischen 2 bis 10 A/cm² gehalten werden, damit die entstehenden Gasblasen den Abtragsprozess unterstützen, indem sie abgetragenes Material aus den Vertiefungen 11 auswerfen.

Die Vorteile einer Kochsalzlösung als Elektrolyt bestehen darin, dass eine relativ einfache Aufbereitung vom Elektrolyten durch Filtrieren möglich ist, dass der Elektrolyt einfach herzustellen ist, und dass der Elektrolyt keinerlei Risiken für das Bedienungspersonal oder den späteren Träger des Implantats in sich birgt.

Die Elektroden 26 können aus Kohle oder Metall hergestellt werden, wobei Messing vorgezogen wird, weil es leicht zu bearbeiten ist und beim elektrochemischen Abtragen in Kochsalzlösung nicht korrodiert.

In Figur 7b ist die Gegenelektrode 26 ebenfalls ortsfest an einem Ständer 55 befestigt. Werkstück 1 und Gegenelektrode 26 sind über Zuleitungen 53 an eine Spannungsquelle in einer Steuerung 43 zuschaltbar. Stromund Spannungswerte sind einstellbar und können an Anzeigen 54 abgelesen werden.

In Figur 7c ist eine Gegenelektrode 26 gezeigt, die im vorgesehenen Abtragsbereich einen engen Spalt zum Werkstück 1 aufweist und über viele Bohrungen 77 frischen Elektrolyten in den Arbeitsspalt einspritzt. Die Bohrungen 77 enden in einem Hohlraum 78, der über ein Spülrohr 51 mit frischem Elektrolyten 52 versorgt wird.

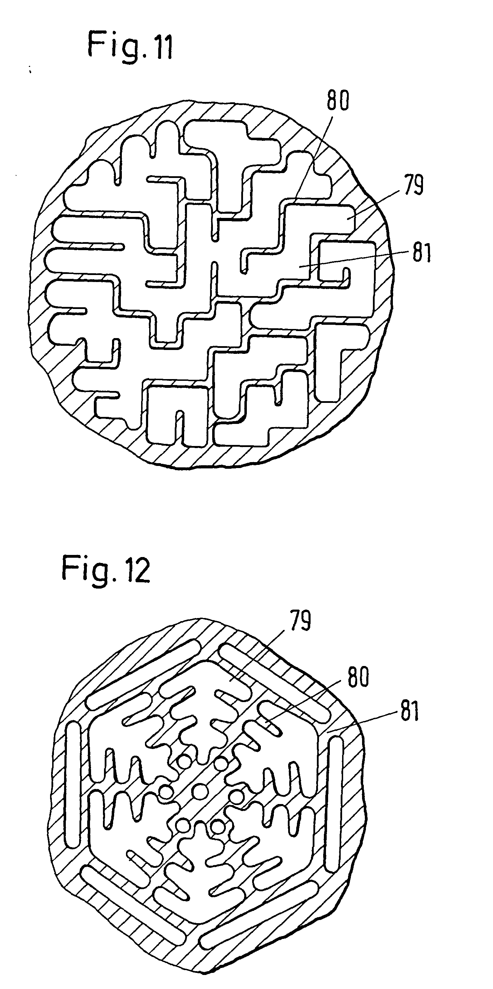

Zwei weitere Beispiele für die Gestaltung von Implantatsoberflächen sind in den Figuren 11 und 12 gezeigt. Mit dem Laser geschriebene Furchenabschnitte im Schutzlack lassen mit dem nachfolgenden elektrochemischen Abtragen Rinnenabschnitte 79 entstehen, die sich mehr oder weniger durchdringen und die durch stehengebliebene Rippen 80 voneinander getrennt sind. Durch die grösseren zusammenhängenden Flächen wird hier die Blutversorgung vom einsprossenden Gewebe verbessert. Längs der Rinnenabschnitte 79 können grössere Blutgefässe entstehen. In Figur 11 ist ein wirres orthogonales Netz als Teilfläche 81 gezeigt, die durch Wiederholung grössere Bereiche abdecken kann. In Figur 12 ist eine als Sechseck aufgebaute Teilfläche 81 mit tannenbaumähnlichen Strukturen gezeigt, die das Einwachsen ebenfalls erleichtern. Teilflächen dieser Art haben den Vorteil, dass sie beim Schreiben mit dem Laser auch auf gekrümmten Flächen aneinandergereiht werden können, ohne dass eine zu grosse Verzerrung entsteht.

Das präzise Schreiben von Furchenabschnitten lässt sich am einfachsten mit einer mehrachsigen numerischen Steuerung bewältigen, die die Relativbewegung zwischen Implantat und Laser beim Schreiben vorgibt.

Die hier beschriebenen Oberflächen und die Verfahren für ihre Herstellung lassen sich auch ganz allgemein auf anderen Gebieten anwenden, bei denen ein metallisches Substrat mit einer Struktur aus Erhöhungen und Vertiefungen versehen wird, um eine bessere Verbindung zu einem anderen Werkstoff herzustellen. So lassen sich die Lagerschalen von Gleitlagern mit einer solchen Struktur versehen, bevor der eigentliche Gleitwerkstoff aufgebracht wird, sei es, dass der Gleitwerkstoff aufgepresst wird wie beispielsweise Teflon ™, sei es, dass der Gleitwerkstoff metallisch aufgespritzt wird.

Ebenso ist es möglich die metallische Oberfläche von Presswerkzeugen, Spritzwerkzeugen oder Walzen mit dieser Grobstruktur zu versehen, welche mittels Formumkehr auf andere Teile beispielsweise aus Kunststoff als Negativ übertragbar ist.

Am Verfahren selbst lassen sich die einzelnen Verfahrensschritte durch zusätzliche Massnahmen verbessern. So kann beim elektrochemischen Abtragen die Wirkung einer Salzlösung verbessert werden, indem sie auf einer konstanten und leicht erhöhten Temperatur, die zwischen 50°C und dem Siedepunkt liegt, gehalten wird. Im weiteren kann der Abtrag durch schnelles Bewegen des Werkstücks in der Abtragsflüssigkeit verbessert werden, indem zum Beispiel das Werkstück wie eine Sonotrode in einem Ultraschallkopf während des Abtragens eingespannt ist. 25 kH_{z} ist eine typische Frequenz für einen Ultraschallschwinger, der ein Werkstück mit Grösse eines künstlichen Hüftgelenkschaftes bewegt. Es genügt aber auch, das Werkstück mit einer Frequenz von 50-150 H_{z} zu rütteln, indem man einen Schwingungserreger an der Aufhängung des Werkstücks angreifen lässt.

Die hier mit ihren Handelsnamen bezeichneten Produkte sind in der Zusammensetzung zu verstehen, in der sie bis zum Zeitpunkt der Patentanmeldung im Handel erhältlich waren.

## Patentansprüche

1. Metallimplantat beispielsweise für Knochen mit einer Oberfläche (2), die eine Grobstruktur aus Erhöhungen (3) und Vertiefungen (4) aufweist, **dadurch gekennzeichnet, dass** die Oberfläche von einem Netz (5) von vorstehenden Rippen (6) durchzogen ist, welche Knotenpunkte (7) und Maschen (8) mit einer Maschenweite (9) von 2 mm bis 0,4 mm bilden, dass die Maschen (8) im wesentlichen jeweils eine Vertiefung in Form eines Ausschnitts aus einer Kugelkalotte (11) bilden und dass die Grobstruktur eine Feinstruktur für die Verankerung aufweist.

2. Implantat nach Anspruch 1, **dadurch gekennzeichnet, dass** die Kugelkalotten (11) eine Tiefe (12) gegenüber den Knotenpunkten (7) aufweisen, die zwischen 10 % und 60 % des Durchmessers (30) der Kugelkalotte (11) liegt.

3. Implantat nach Anspruch 1 oder 2, **dadurch gekennzeichnet, dass** sich benachbarte Kugelkalotten (11) an ihren Rändern teilweise durchdringen, um Rippen (6) mit Sätteln (10) zu bilden, während die Knotenpunkte (7) der Rippen (6) wie Berggipfel weiter vorstehen.

4. Implantat nach Anspruch 3, **dadurch gekennzeichnet, dass** die Sattelpunkte (10) der Rippen (6) mit einer Absenkung V von weniger als 50 % der Tiefe (12) der Kugelkalotten (11) gegenüber den Knotenpunkten (7) zurückstehen.

5. Implantat nach einem der Ansprüche 1 bis 4, **dadurch gekennzeichnet, dass** die Kugelkalotten (11) mit ihren Mittelpunkten in Abständen (17) angeordnet sind, die regelmässigen Rasterabständen entsprechen.

6. Implantat nach einem der Ansprüche 1 bis 5, **dadurch gekennzeichnet, dass** die Oberfläche (2) aus Titan oder aus einer Titanlegierung besteht.

7. Verfahren zum Herstellen einer Oberfläche (2), an einem Implantat (1) aus Metall nach einem der Ansprüche 1 bis 6,
wobei die Metalloberfläche mit einem elektrochemisch wirksamen Schutzlack (15) abgedeckt wird, welcher sie später vor Abtrag in einer elektrochemischen Flüssigkeit schützt, **dadurch gekennzeichnet, dass** Löcher (18) mit einem kleineren Durchmesser (19) als die Maschenweite (9) in einem Abstand (17) der späteren Maschen (8) im Schutzlack (15) ausgespart werden,
dass die mit Löchern im Schutzlack versehene Oberfläche zeitlich begrenzt einer elektrolytischen Flüssigkeit (20) ausgesetzt wird, um durch elektrochemisches Abtragen oder Aetzen Ausschnitte von Kugelkalotten (11) zu erzeugen, die durch schmale Rippen (6) voneinander getrennt sind und eine grob strukturierte Zwischenoberfläche (14) bilden,
dass der restliche Schutzlack (15) entfernt wird und dass die grobstrukturierte Oberfläche (14) mit einer Feinstruktur, beispielsweise durch Sandstrahlen, versehen wird.

8. Verfahren nach Anspruch 7, **dadurch gekennzeichnet, dass** ein Schutzlack aufgetragen wird, der durch Strahlung zur Verfestigung gebracht wird, wobei die späteren Löcher durch Masken ausgespart werden, um dort den unverfestigten Schutzlack für die nachfolgende elektrochemische Bearbeitung auszuwaschen oder durch Erosion zu entfernen.

9. Verfahren zum Herstellen einer Oberfläche, an einem Metallteil, beispielsweise an einem Metallimplantat, die eine Grobstruktur (14) aus einem Netz (5) von vorstehenden Rippen (6) aufweist, welche Knotenpunkte (7) und Maschen (8) mit einer Maschenweite (9) von 2mm bis 0,4mm bilden, wobei die Maschen (8) jeweils in Form eines Ausschnitts einer Kugelkalotte (11) ausgebildet sind, wobei
- in einem ersten Schritt die Metalloberfläche mit einem haftenden, elektrochemisch wirksamen Schutzlack (15) abgedeckt wird, der sich mit einem Laser (13) ausgesuchter Wellenlänge bei so niedriger Energiedichte abtragen lässt, dass eine signifikante Energieschwelle gegenüber der für Metallabtrag notwendigen Energiedichte besteht, die den Metallabtrag ausschliesst,
- in einem weiteren Schritt mit dem Laser (13) ausgesuchter Wellenlänge (16) entsprechend den Abständen (17) der vorgesehenen Kugelkalotten (11) Löcher (18) in den Schutzlack (15) geschossen werden, deren Durchmesser (19) kleiner sind als die Durchmesser (30) der späteren Kugelkalotten (11),
- in einem weiteren Schritt die mit Löchern (18) im Schutzlack (15) versehene Oberfläche einer elektrisch leitenden Flüssigkeit (20) ausgesetzt wird, um zeitlich begrenzt durch elektrochemisches Abtragen und/oder Aetzen, die Kalotten (11) zu erzeugen, welche im Bereich der Löcher (18) durch Abtrag gleichzeitig in die Tiefe und parallel zur Oberfläche wachsen, und
- in einem weiteren Schritt der verbliebene Schutzlack entfernt wird.

10. Verfahren zum Erzeugen einer Oberfläche, an einem Metallteil beispielsweise, an einem Metallimplantat, die eine Grobstruktur aus Erhöhungen in Form von Rippen (79) und aus Vertiefungen in Form von Rinnen mit einer Breite von 2 mm bis 0,4 mm aufweist, wobei
- in einem ersten Schritt die Metalloberfläche mit einem haftenden, elektrochemisch wirksamen Schutzlack abgedeckt wird, der sich mit einem Laser ausgesuchter Wellenlänge bei so niedriger Energiedichte abtragen lässt, dass eine signifikante Energieschwelle gegenüber der für Metallabtrag notwendigen Energiedichte besteht, die den Metallabtrag ausschliesst,
- in einem weiteren Schritt mit dem Laser ausgesuchter Wellenlänge entsprechend den Mittellinien der vorgesehenen Rinnen Furchen in den Schutzlack geschrieben werden, deren Breiten nur einen Bruchteil der Breiten der späteren Rinnen betragen,
- in einem weiteren Schritt die mit Furchen im Schutzlack versehene Oberfläche einer elektrisch leitenden Flüssigkeit ausgesetzt wird, um zeitlich begrenzt durch elektrochemisches Abtragen und/oder Aetzen, die Rinnen zu erzeugen, welche im Bereich der Furchen durch Abtragen gleichzeitig in die Tiefe und parallel zur Oberfläche in die Breite wachsen, und
- in einem weiteren Schritt der verbliebene Schutzlack entfernt wird.

11. Verfahren nach einem der Ansprüche 9 oder 10, **dadurch gekennzeichnet, dass**
- der Schutzlack im Vakuum durch Verdampfen als Monomer auf dem Implantat aufgetragen wird, um ihn zu einem Schutzfilm (15) mit einer Dicke 3 µm < S < 100 µm, vorzugsweise mit einer Dicke 5 µm < S < 10 µm, polymerisieren zu lassen,
- als Laser ein Modell eingesetzt wird, das eine mehr als fünfzigmal geringere Energiedichte als zum Schmelzen und Schweissen von Metall bei einem Brennfleck der Löcher (18) erzeugt, beispielsweise bei einer Lackschicht mit einer Dicke S = 7 µm ein 25 Watt CO₂ Laser, der bei einer Wellenlänge von 10.6 µm eine Energiedichte von 2 J/mm² erzeugt, und
- als elektrisch leitende Flüssigkeit (24) Salzlösungen, Lösungen von Kaliumbromid, von Natriumnitrat, von Natriumchlorat oder verdünnte Säuren verwendet werden, um mit elektrischer Unterstützung elektrochemisch abzutragen oder zu ätzen.

12. Verfahren nach einem der Ansprüche 7, 9 oder 10, **dadurch gekennzeichnet, dass** bei einem Implantat aus Titan oder aus einer Titanlegierung als elektrisch leitende Flüssigkeit eine Kochsalzlösung (25) verwendet wird und dass in der Kochsalzlösung zwischen Implantat und einer Elektrode zeitweise eine elektrische Spannung angelegt wird, um über die Einwirkzeit eine vorgegebene Grösse der an den Löchern (18) entstehenden Kalotten (11) einzuhalten.

13. Verfahren nach einem der Ansprüche 7, 9 oder 10, **dadurch gekennzeichnet, dass** zum elektrochemischen Abtragen eine Mindestspannung < 100 Volt an das Implantat angelegt wird, um passivierende Schichten abzubauen, während der eigentliche Abtrag bei Spannungen zwischen 15 und 60 Volt stattfindet, vorzugsweise für Titan bei einer Spannung von 40 Volt.

14. Verfahren nach einem der Ansprüche 7, 9 oder 10, **dadurch gekennzeichnet, dass** als Schutzlack (15) ein in Methanol lösliches Phenolharz verwendet wird,

15. Verfahren nach einem der Ansprüche 7, 9 oder 10, **dadurch gekennzeichnet, dass** als Schutzlack (15) ein phenolhaltiges Epoxidharz verwendet wird,

16. Verfahren nach einem der Ansprüche 7, 9 oder 10, **dadurch gekennzeichnet, dass** der Schutzlack (15) durch elektrostatisches Pulverbeschichten aufgebracht wird.

17. Verfahren nach Anspruch 7, **dadurch gekennzeichnet, dass** nach dem Aetzen oder elektrochemischen Abtragen die stehengebliebene Schutzlackschicht (15) bei Temperaturen unter 400°C rissig versprödet wird, beispielsweise durch Heissluft oder Infrarotwärme, um sie anschliessend mechanisch beispielsweise durch Sandstrahlen oder durch eine Aetzlauge abzutragen.

18. Verfahren nach einem der Ansprüche 9 oder 10, **dadurch gekennzeichnet, dass** ein Laser mit einer Wellenlänge grösser als 2 µm verwendet wird, um teilweise eine Reflexion an der Metalloberfläche zu erreichen.

19. Verfahren nach einem der Ansprüche 7 oder 9, **dadurch gekennzeichnet, dass** zur Erzeugung unterschiedlicher Tiefen (12) bei einem bestimmten Durchmesser (9) der Kugelkalotten (11) an Zwischenoberflächen (14) einerseits der Durchmesser (19) der Löcher (18) im Schutzlack (15) klein gehalten wird und die Aetzzeit vergrössert wird, um tiefe halbkugelförmige Kalotten (11) zu erzeugen oder andererseits der Duchmesser (19) der Löcher (18) im Schutzlack (15) gross gehalten wird und die Aetzzeit verkleinert wird, um flache Ausschnitte von Kugelkalotten (11) zu erhalten.

20. Verfahren nach einem der Ansprüche 7, 9 oder 10, **dadurch gekennzeichnet, dass** beim elektrochemischen Abtragen eine flächige Elektrode (26) in einem Abstand zwischen 1 mm bis 10 mm zum Implantat angebracht ist und mindestens einen Teil des Implantats (1) mit ihrer Fläche (27) abdeckt, um einen Kanal (28) zu bilden, wobei die Stromdichte quer über den Kanal kleiner als 20 A/cm² vorzugsweise zwischen 2 und 10 A/cm² gehalten wird.

21. Verfahren nach Anspruch 20, **dadurch gekennzeichnet, dass** die Abtragsflüssigkeit im Kanal (28) von einer Spüleinrichtung mit einer Geschwindigkeit im Kanal von 100 bis 400 mm/s ausgewechselt wird.

22. Verfahren nach Anspruch 20 oder 21, **dadurch gekennzeichnet, dass** bei rotationssymmetrischen Implantaten wie zum Beispiel Hüftgelenkschalen das Implantat (1) in der Flüssigkeit (20) um seine Rotationsachse (29) gedreht wird, während die Elektrode (26) nur einen Teil der zu bearbeitenden Fläche des Implantats abdeckt.

23. Verfahren nach einem der Ansprüche 7, 9 oder 10, **dadurch gekennzeichnet, dass** zur besseren Regelung der Stromdichte im Elektrolyten (20) dessen Temperatur einigermassen konstant gehalten wird, beispielsweise für eine Kochsalzlösung bei einer Temperatur < 90°C.

24. Verfahren nach einem der Ansprüche 7 bis 23, **dadurch gekennzeichnet, dass** als vorbereitende oder Zwischenschritte Reinigungsoperationen vorgesehen sind.

## Claims

1. Metal implant, for example for bones, with a surface (2) which has a coarse structure of elevations (3) and depressions (4) **characterised in that** the surface is covered by a network (5) of protruding ribs which form nodes (7) and interstices (8) having an interstice width of 2 mm to 0.4 mm; **in that** the interstices (8) each essentially form a recess in the form of a section of a spherical cavity (11); and **in that** the coarse structure has a fine structure for the anchoring.

2. Implant in accordance with claim 1, **characterised in that** the spherical cavities (11) have a depth (12) with respect to the nodes (7) which lies between 10 % and 60 % of the diameter (30) of the spherical cavity (11).

3. Implant in accordance with claim 1 or claim 2, **characterised in that** adjacent spherical cavities (11) partially penetrate one another at their edges in order to form ribs (6) with saddles (10), whereas the nodes (7) of the ribs (6) protrude further like mountain peaks.

4. Implant in accordance with claim 3, **characterised in that** the saddle points (10) of the ribs (6) are recessed with respect to the nodes (7) by an indentation V of less than 50 % of the depth (12) of the spherical cavities (11).

5. Implant in accordance with one of the claims 1 to 4, **characterised in that** the mid-points of the spherical cavities (11) are arranged at distances (17) which correspond to regular grid spacings.

6. Implant in accordance with one of the claims 1 to 5, **characterised in that** the surface (2) consists of titanium or a titanium alloy.

7. Method for producing a surface (2) on an implant (1) of metal in accordance with one of the claims 1 to 6, wherein the metal surface is covered over with an electrochemically active protective lacquer (15) which later protects it from erosion in an electrochemical liquid, **characterised in that** holes (18) with a smaller diameter (19) than the interstice width (9) are left or formed at a spacing (17) of the later interstices (8);
**in that** the surface which is provided with holes in the protective lacquer is exposed to an electrolytic liquid (20) for a limited time in order to produce sections of spherical cavities (11) by electrochemical erosion or etching which are separated from one another by narrow ribs (6) and a coarsely structured intermediate surface (14);
**in that** the remaining protective lacquer (15) is removed; and **in that** the coarsely structured surface (14) is provided with a fine structure, for example by means of sand blasting.

8. Method in accordance with claim 7, **characterised in that** a protective lacquer is applied which is caused to harden through irradiation, with the later holes being formed by means of masks, in order to wash out or remove by erosion the unhardened protective lacquer for subsequent electrochemical processing.

9. Method for the manufacture of a surface on a metal part, for example on a metal implant, having a coarse structure (14) of a network (5) of projecting ribs (6) which form nodes (7) and interstices (8) with an interstice width (9) from 2 mm to 0.4 mm and with the interstices (8) each having the form of a section of a spherical cavity (11), wherein
- in a first step, the metal surface is coated over with an adherent, electrochemically active protective lacquer or coating (15) which can be eroded by a laser (13) of selected wavelength at such a low energy density that there is a significant energy threshold with respect to the energy density required for metal erosion, which precludes metal erosion,
- in a further step holes are shot into the protective lacquer (15) using the laser (13) of selected wavelength (16), in accordance with the spacings (17) of the envisaged spherical cavities (11), with the diameters (19) of the holes (18) being smaller than the diameters of the later spherical cavities (11),
- in a further step, the surface which is provided with holes (18) in the protective lacquer (15) is exposed to an electrically conducting liquid (20) in order to produce the spherical cavities (11) with time limitation by electrochemical erosion and/or etching which grow simultaneously in depth and parallel to the surface in the region of the holes (18), and
- the remaining protective lacquer is removed in a further step.

10. Method for the production of a surface on a metal part, for example on a metal implant, having a coarse structure of elevations in the form of ribs (79) and depressions in the form of channels with a width of 2 mm to 0.4 mm, wherein
- in a first step, the metal surface is coated over with an adherent, electrochemically active protective lacquer or coating which can be eroded by a laser of selected wavelength at such a low energy density that there is a significant energy threshold with respect to the energy density required for metal erosion, which precludes metal erosion,
- in a further step grooves are inscribed in the protective lacquer in accordance with the centrelines of the envisaged channels using the laser of selected wavelength, with the widths of the grooves only amounting to a fraction of the widths of the later channels,
- in a further step, the surface which is provided with grooves in the protective lacquer is exposed to an electrically conducting liquid in order to produce the channels with time limitation by electrochemical erosion and/or etching which grow simultaneously in depth and parallel to the surface in the region of the grooves, and
- the remaining protective lacquer is removed in a further step.

11. Method in accordance with one of the claims 9 or 10, **characterised in that**
- the protective lacquer is applied onto the implant
in the vacuum as a monomer by vaporisation in order to allow it to polymerise to a protective film (15) with a thickness 3 µm < S < 100 µm, preferably with a thickness 5 µm < S < 10 µm.
- a model of laser is used which produces a more than 50 times lower energy density at a focal spot of the holes (18) than for the melting and welding of metal, for example, for a protective layer with a thickness S = 7 µm, a 25 watt CO₂ laser which produces an energy density of 2 J/mm² at a wavelength of 10.6 µm, and
- salt solutions, solutions of potassium bromide, of sodium nitrate, of sodium chlorate or dilute acids are used as the electrically conducting liquid (24) in order to erode or to etch electrochemically with electrical assistance.

12. Method in accordance with one of the claims 7, 9 or 10, **characterised in that** a table salt solution (25) is used as the electrically conducting liquid for an implant of titanium or of a titanium alloy; and **in that** an electrical voltage is applied in the salt solution between the implant and an electrode for a period of time in order to achieve a predetermined size of the spherical cavities (11) arising at the holes (18) via the time of action.

13. Method in accordance with one of the claims 7, 9 or 10, **characterised in that** a minimum voltage < 100 volts is applied to the implant for the electrochemical erosion in order to remove passivating layers, whereas the actual erosion takes place at voltages between 15 and 60 volts, for titanium preferably at a voltage of 40 volts.

14. Method in accordance with one of the claims 7, 9 or 10, **characterised in that** a phenol resin soluble in methanol is used as the protective lacquer (15).

15. Method in accordance with one of the claims 7, 9 or 10, **characterised in that** an epoxy resin containing phenol is used as the protective lacquer (15).

16. Method in accordance with one of the claims 7, 9 or 10, **characterised in that** the protective lacquer (15) is applied by electrostatic powder coating.

17. Method in accordance with claim 7 **characterised in that** after the etching or electrochemical erosion the residual protective lacquer coating (15) is embrittled in a fissured manner at temperatures less than 400°C, by hot air or infrared heat for example, in order to subsequently remove it mechanically, for example by means of sandblasting or by a caustic etch.

18. Method in accordance with one of the claims 9 to 17, **characterised in that** a laser with a wavelength greater than 2 µm is used in order to partly achieve a reflection at the metal surface.

19. Method in accordance with one of the claims 7 or 9, **characterised in that**, for the production of different depths (12) with a given diameter (9) of the spherical cavities (11) at intermediate surfaces (14), either, on the one hand, the diameter (19) of the holes (18) in the protective lacquer (15) is kept small and the etching time is lengthened in order to produce deep hemispherical cavities (11), or, on the other hand, the diameter (19) of the holes (18) in the protective lacquer (15) is kept large and the etching time is shortened in order to obtain shallow sections of spherical cavities (11).

20. Method in accordance with one of the claims 7, 9 or 10, **characterised in that** during the electrochemical erosion an areal electrode (26) is arranged at a distance of between 1 mm to 10 mm from the implant and covers at least a part of the implant (1) with its surface (27) in order to form a channel (28), with the current density across the channel being kept less than 20 A/cm², preferably between 2 and 10 A/cm².

21. Method in accordance with claim 20 **characterised in that** the erosion liquid in the channel (28) is replaced by a flushing apparatus with a speed in the channel of 100 to 400 mm/s.

22. Method in accordance with claim 20 or 21, **characterised in that**, for rotationally symmetrical implants such as hip joint shells for example, the implant (1) is rotated in the liquid (20) about its axis of rotation (29), while the electrode (26) only covers a part of the surface of the implant to be processed.

23. Method in accordance with one of the claims 7, 9 or 10, **characterised in that**, for the improved regulation of the current density in the electrolyte (20), its temperature is held constant to a certain extent, for example, at a temperature < 90°C for a cooking salt solution.

24. Method in accordance with one of the claims 7 to 23, **characterised in that** cleaning operations are provided as preparatory or intermediate steps.

## Revendications

1. Implant métallique, par exemple pour des os, avec une surface (2) qui présente une structure grossière constituée de reliefs (3) et de creux (4), **caractérisé en ce que** la surface est traversée par un réseau (5) de nervures saillantes (6) qui forment des noeuds (7) et des mailles (8) d'une largeur de maille (9) de 2 mm à 0,4 mm, **en ce que** les mailles (8) forment sensiblement respectivement un creux sous la forme d'une découpure d'une calotte sphérique (11) et **en ce que** la structure grossière présente une structure fine pour l'ancrage.

2. Implant selon la revendication 1, **caractérisé en ce que** les calottes sphériques (11) présentent une profondeur (12) par rapport aux noeuds (7) qui se situe entre 10% et 60% du diamètre (30) de la calotte sphérique (11).

3. Implant selon la revendication 1 ou 2, **caractérisé en ce que** des calottes sphériques avoisinantes (11) s'interpénètrent partiellement à leurs bords pour former des nervures (6) avec des selles (10) tandis que les noeuds (7) des nervures (6) font saillie davantage, comme des sommets de montagnes.

4. Implant selon la revendication 3, **caractérisé en ce que** les points de selle (10) des nervures (6) sont en retrait avec un abaissement V inférieur à 50% de la profondeur (12) des calottes sphériques (11) par rapport aux noeuds (7).

5. Implant selon l'une des revendications 1 à 4, **caractérisé en ce que** les calottes sphériques (11) sont disposées avec leurs centres selon des écarts (17) qui correspondent à des écarts de grille réguliers.

6. Implant selon l'une des revendications 1 à 5, **caractérisé en ce que** la surface (2) est constituée de titane ou d'un alliage de titane.

7. Procédé de fabrication d'une surface (2), à un implant (1) en métal selon l'une des revendications 1 à 6,
où la surface métallique est recouverte d'un vernis de protection (15) efficace d'une manière électrochimique qui la protège ultérieurement à l'encontre d'une usure dans un liquide électrochimique, **caractérisé en ce que** des trous (18) d'un plus petit diamètre (19) que la largeur de maille (9) sont évidés à un écart (17) des mailles ultérieures (8) dans le vernis de protection (15),
**en ce que** la surface présentant des trous dans le vernis de protection est exposée d'une manière limitée dans le temps à un liquide électrolytique (20) pour produire par enlèvement ou attaque électrochimique des découpures de calottes sphériques (11) qui sont séparées par des nervures étroites (6) les unes des autres et qui forment une surface intermédiaire (14) structurée grossièrement,
**en ce que** le vernis de protection restant (15) est enlevé et **en ce que** la surface structurée grossièrement (14) est pourvue d'une structure fine, par exemple par décapage au sable.

8. Procédé selon la revendication 7, **caractérisé en ce qu'**un vernis de protection est appliqué qui est amené à durcir par rayonnement, où les trous ultérieurs sont évidés par des masques pour y éliminer par lavage ou enlever par érosion le vernis de protection non solidifié en vue du traitement électrochimique subséquent.

9. Procédé de fabrication d'une surface, à une pièce métallique, par exemple à un implant métallique, qui présente une structure grossière (14) en un réseau (5) de nervures saillantes (6) qui forment des noeuds (7) et des mailles (8) d'une largeur de maille (9) de 2 mm à 0,4 mm, où les mailles (8) sont réalisées chacune sous la forme d'une découpure d'une calotte sphérique (11), où
- lors d'une première étape, la surface métallique est recouverte d'un vernis de protection adhérent (15), efficace d'une manière électrochimique qui peut être enlevé par un laser (13) d'une longueur d'onde sélectionnée à une densité d'énergie tellement basse qu'il existe un seuil d'énergie significatif par rapport à la densité d'énergie requise pour l'enlèvement du métal qui exclut l'enlèvement du métal,
- lors d'une étape ultérieure, avec le laser (13) d'une longueur d'onde sélectionnée (16), conformément aux écarts (17) des calottes sphériques prévues (11), des trous (18) sont tirés dans le vernis de protection (15) dont les diamètres (19) sont plus petits que les diamètres (30) des calottes sphériques ultérieures (11),
- lors d'une étape ultérieure, la surface pourvue de trous (18) dans le vernis de protection (15) est exposée à un liquide (20) électriquement conducteur pour produire d'une manière limitée dans le temps, par enlèvement et/ou attaque électrochimique, les calottes (11) qui croissent au voisinage des trous (18) en raison de l'enlèvement en même temps en profondeur et parallèlement à la surface et
- lors d'une étape ultérieure, le vernis de protection restant est enlevé.

10. Procédé de production d'une surface, à une pièce métallique, par exemple à un implant métallique, qui présente une structure grossière en reliefs sous la forme de nervures (79) et de creux sous la forme de rainures d'une largeur de 2 mm à 0,4 mm, où
- lors d'une première étape, la surface métallique est recouverte d'un vernis de protection adhérent, efficace d'une manière électrochimique, qui peut être enlevé avec un laser d'une longueur d'onde sélectionnée à une densité d'énergie tellement basse qu'il existe un seuil d'énergie significatif par rapport à la densité d'énergie requise pour l'enlèvement du métal qui exclut l'enlèvement du métal,
- lors d'une étape suivante, au moyen du laser de la longueur d'onde sélectionnée, conformément aux lignes médianes des rainures prévues, des sillons sont tracés dans le vernis de protection dont les largeurs représentent seulement une fraction des largeurs des rainures ultérieures,
- lors d'une étape ultérieure, la surface pourvue de sillons dans le vernis de protection est exposée à un liquide électriquement conducteur pour produire d'une manière limitée dans le temps par enlèvement et/ou attaque électrochimique les rainures qui croissent au voisinage des sillons par enlèvement en même temps en profondeur et parallèlement à la surface en largeur et,
- lors d'une étape ultérieure, le vernis de protection restant est enlevé.

11. Procédé selon l'une des revendications 9 ou 10, **caractérisé en ce que**
- le vernis de protection est appliqué sous vide par évaporation comme monomère sur l'implant pour le laisser polymériser en un film de protection (15) d'une épaisseur de 3 µm < S < 100 µm, de préférence en une épaisseur de 5 µm < S < 10 µm,
- comme laser, un modèle est utilisé qui produit une densité d'énergie plus que cinquante fois plus réduite que pour la fusion et le soudage de métal à un foyer des trous (18), par exemple dans une couche de vernis d'une épaisseur S = 7 µm, un laser CO₂ de 25 Watt qui produit à une longueur d'onde de 10,6 µm, une densité d'énergie de 2 J/mm², et
- comme liquide (24) électriquement conducteur, des solutions salines, des solutions de bromure de potassium, de nitrure de sodium, de chlorure de sodium ou des acides dilués sont utilisés pour enlever ou attaquer d'une manière électrochimique avec un soutien électrique.

12. Procédé selon l'une des revendications 7, 9 ou 10, **caractérisé en ce que** dans le cas d'un implant en titane ou en un alliage de titane, du chlorure de sodium (25) est utilisé comme liquide électriquement conducteur et **en ce qu'**il est appliqué dans le chlorure de sodium entre l'implant et une électrode temporairement une tension électrique pour conserver pendant le temps d'action une grandeur prédéterminée des calottes (11) produites aux trous (18).

13. Procédé selon l'une des revendications 7, 9 ou 10, **caractérisé en ce que** pour l'enlèvement électrochimique, une tension minimale < 100 volts est appliquée à l'implant pour enlever des couches passivantes tandis que l'enlèvement proprement dit a lieu à des tensions entre 15 et 60 volts, de préférence pour le titane à une tension de 40 volts.

14. Procédé selon l'une des revendications 7, 9 ou 10, **caractérisé en ce qu'**une résine phénolique soluble dans du méthanol est utilisée comme vernis de protection (15).

15. Procédé selon l'une des revendications 7, 9 ou 10, **caractérisé en ce qu'**une résine époxy contenant du phénol est utilisée comme vernis de protection (15).

16. Procédé selon l'une des revendications 7, 9 ou 10, **caractérisé en ce que** le vernis de protection (15) est appliqué par revêtement électrostatique par poudre.

17. Procédé selon la revendication 7, **caractérisé en ce qu'**après l'attaque ou l'enlèvement électrochimique, la couche de vernis de protection subsistante (15) est fragilisée de façon à former des fissures à des températures inférieures à 400°C, par exemple par de l'air chaud ou de la chaleur infrarouge pour l'enlever ensuite mécaniquement, par exemple par un décapage au sable ou par une lessive d'attaque.

18. Procédé selon l'une des revendications 9 ou 10, **caractérisé en ce qu'**un laser d'une longueur d'onde supérieure à 2 µm est utilisé pour obtenir partiellement une réflexion à la surface métallique.

19. Procédé selon l'une des revendications 7 ou 9, **caractérisé en ce que**, pour produire des profondeurs différentes (12), lors d'un diamètre déterminé (9) des calottes sphériques (11) à des surfaces intermédiaires (14), d'une part, le diamètre (19) des trous (18) dans le vernis de protection (15) est maintenu petit et le temps d'attaque est allongé pour produire des calottes demi-sphériques profondes (11) ou bien, d'autre part, le diamètre (19) des trous (18) dans le vernis de protection (15) est maintenu grand et le temps d'attaque est raccourci pour obtenir des découpures plates de calottes sphériques (11).

20. Procédé selon l'une des revendications 7, 9 ou 10, **caractérisé en ce que** lors de l'enlèvement électrochimique, une électrode plane (26) est disposée à un écart entre 1 mm à 10 mm de l'implant et recouvre au moins une partie de l'implant (1) avec sa face (27) pour former un canal (28), où la densité du courant à travers le canal est maintenue plus petite que 20A/cm², de préférence entre 2 et 10A/cm².

21. Procédé selon la revendication 20, **caractérisé en ce que** le liquide d'enlèvement dans le canal (28) est échangé par une installation de rinçage à une vitesse dans le canal de 100 à 400 mm/s.

22. Procédé selon la revendication 20 ou 21, **caractérisé en ce que** dans des implants symétriques en rotation, comme par exemple des coques d'articulation de la hanche, l'implant (1) est amené à tourner dans le liquide (20) autour de son axe de rotation (29) pendant que l'électrode (26) recouvre seulement une partie de la face à façonner de l'implant.

23. Procédé selon l'une des revendications 7, 9 ou 10, **caractérisé en ce qu'**en vue d'un meilleur réglage de la densité du courant dans l'électrolyte (20), la température de celui-ci est maintenue à un niveau approximativement constant, par exemple pour une solution de chlorure de sodium à une température < 90°C.

24. Procédé selon l'une des revendications 7 à 23, **caractérisé en ce que** des opérations de nettoyage sont prévues comme étapes préparatoires ou intermédiaires.
